(19) Europäisches Patentamt
European Patent Office
Office européen des brevets

(11) Publication number: 0 154 495
A2

(12) # EUROPEAN PATENT APPLICATION

(21) Application number: 85301284.7

(22) Date of filing: 26.02.85

(51) Int Cl.⁴: C 07 H 17/08
A 61 K 31/70

(30) Priority: 27.02.84 GB 8405087

(43) Date of publication of application: 11.09.85 Bulletin 85/37

(84) Designated Contracting States: AT BE CH DE FR GB IT LI NL SE

(71) Applicant: Omura, Satoshi
12-7 Seta 5-chome
Setagaya-Ku Tokyo(JP)

(72) Inventor: Omura, Satoshi
12-7, Seta 5-chome
Setagaya-ku Tokyo(JP)

(72) Inventor: Tsuzuki, Kazuo
18-22, Nishiazabu 4-chome
Minato-ku Tokyo(JP)

(74) Representative: Hudson, Christopher Mark et al,
Erl Wood Manor
Windlesham Surrey GU20 6PH(GB)

(54) **Improvements in or relating to thioether macrolide derivatives.**

(57) 11-Thioether and certain C-20 acetal and thioacetal derivatives of tylosin and demycarosyltylosin are effective antibacterial agents.

EP 0 154 495 A2

# IMPROVEMENTS IN OR RELATING TO THIOETHER MACROLIDE DERIVATIVES

This invention relates to macrolide antibiotics, in particular to compounds similar to the well-known macrolide antibiotic tylosin (see, for example Tetrahedron Letters, 2339 (1970) and U.S. Patent Specification No. 3,178,341).

Despite tylosin's great value, there exists a constant and continuing need to develop new antibiotics of this type, both in view of the possibility of emergence of resistant strains, and with a view to improvement in the quantum and/or spectrum of antibiotic activity.

Numerous attempts have been made to modify the tylosin nucleus so as to produce new antibiotics of value, see for instance U.S. Patent Specifications Nos. 4,321,361 and 4,334,019, but thus far none of those modified tylosin derivatives have reached the market place.

According to the present invention there is provided a compound of formula (I):

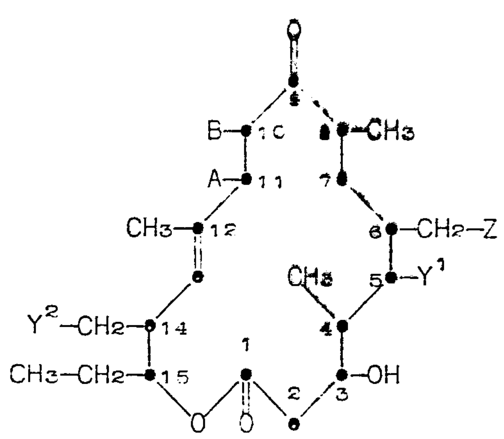

(I)

wherein A represents a group of formula $-SR^1$ where $R^1$ is a $C_{1-6}$ alkyl or $C_{2-6}$ hydroxyalkyl group or a monovalent aryl group when B represents a hydrogen atom;

or A and B taken together represent a single chemical bond;

wherein $Y^1$ represents a group of formula:

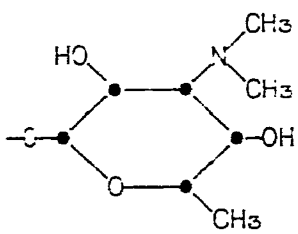

or

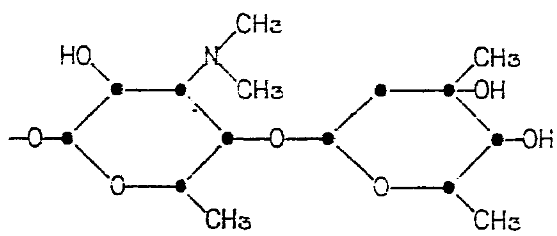

wherein $Y^2$ represents a group of formula:

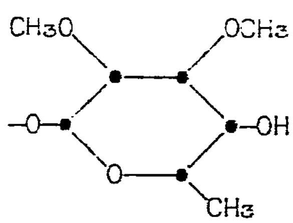

and Z represents CHO or a group of formula:

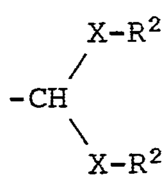

where X represents an oxygen or sulfur atom and $R^2$ is $C_{1-3}$ alkyl or aryl;

provided that when A and B taken together represent a single chemical bond then Z cannot be -CHO and X cannot be oxygen;

or a pharmacologically-acceptable salt thereof.

Although no stereochemical configuration is indicated in the above structural formula, it is to be understood that the stereochemistry is identical to that of tylosin. The $Y^2$ sugar is mycinose and the $Y^1$ sugars are mycaminose and mycarose.

The term "$C_{1-6}$ alkyl group" as used herein includes both straight and branched alkyl groups, for example, methyl, ethyl, propyl, isopropyl, n-butyl, t-butyl, pentyl and hexyl. The term "$C_{2-6}$ hydroxyalkyl" includes groups such as hydroxyethyl, hydroxypropyl and hydroxybutyl.

The term "aryl group" as used herein refers to a monovalent aromatic hydrocarbon group, preferably, to an optionally substituted phenyl group. The aryl, preferably phenyl, group may be substituted by a halogen atom such as chlorine or bromine, an amino group, a $C_{1-4}$ alkyl group such as methyl, ethyl, propyl, n-butyl or t-butyl, or a $C_{1-4}$ alkoxy group such as methoxy, ethoxy, propoxy, n-butoxy or t-butoxy. Such substituted aryl groups may include for instance, halophenyl groups, such as chlorophenyl or bromophenyl, $C_{1-4}$ alkylphenyl groups such as tolyl, xylyl, ethylphenyl or butylphenyl or $C_{1-4}$ alkoxyphenyl groups, such as methoxyphenyl, ethoxyphenyl, propoxyphenyl, n-butoxyphenyl or t-butoxyphenyl. The preferred $R^2$ aryl group is phenyl.

The term "$C_{1-3}$ alkyl group" as used herein means methyl, ethyl, n-propyl or i-propyl.

Preferred compounds of the invention are those wherein Z is -CHO and A is $-SR^1$ where $R^1$ is an optionally substituted phenyl group.

The compounds of formula (I) can be prepared by reacting tylosin or demycarosyltylosin with a compound of formula $R^2OH$, $R^2SH$ or $(R^2)_2S$, where $R^2$ is as defined above, to form a compound of formula (IA):

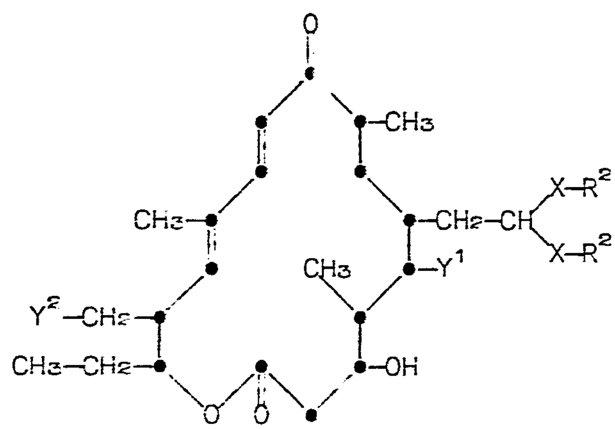

(IA)

In this reaction, when X represents oxygen, it is preferred that $R^2$ be alkyl, when X represents sulfur, it is preferred that $R^2$ be aryl.

Compounds of formula (I) in which A represents $-SR^1$ and B represents hydrogen, and Z is an acetal

or thioacetal group, can be prepared by reacting the compound of formula (IA) with a thiol of formula:

$R^1$-SH

where $R^1$ is as previously defined. This thioetherification can be carried out using Michael-type conditions.

The acetal or thioacetal group:

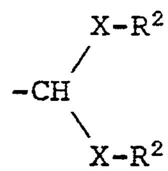

can then be transformed by hydrolysis to yield compounds of formula (I) in which Z is -CHO. If necessary, hydrolysis products of formula (I) can be converted to the corresponding pharmacologically acceptable salt by conventional means.

The tylosin acetal of formula (I) in which X is an oxygen atom and $Y^1$ is mycarosylmycaminosyl may be prepared by reacting tylosin with a hydroxy derivative of formula $R^2OH$ such as an alkanol or a phenol. The alkanol may be methanol, ethanol or propanol. The phenol may be, for instance, phenol, a halogen-substituted phenol such as chlorophenol or bromophenol, a $C_{1-4}$ alkyl-substituted phenol such as cresol, xylenol or ethylphenol or a $C_{1-4}$ alkoxy-substituted phenol such as methoxyphenol or ethoxyphenol. The acetalization may be carried out by using the alkanol to be acetalized in excess as the solvent. As

other solvents there may be used a chlorinated hydrocarbon such as chloroform, benzene, acetonitrile or an ether such as tetrahydrofuran.

The demycarosyltylosin acetal of formula I in which X is an oxygen atom and $Y^1$ is a mycaminosyl group, may be prepared by reacting demycarosyltylosin under substantially the same conditions as used for the preparation of the tylosin acetal. The demycarosyltylosin acetal also may be prepared directly from tylosin by acetalization, if the acetalization is carried out under anhydrous conditions using a mineral acid. In such a circumstance, simultaneous acetalization of the C-20 aldehyde group will occur with simultaneous cleavage of the mycarosyl sugar from the C-5 moiety. As examples of mineral acids suitable for use in this reaction there may be mentioned hydrochloric acid and sulfuric acid. Concentrations of the mineral acid may appropriately range from about 0.2 to 1%. In order to adjust the acid concentration, acidic methanol, typically hydrogen chloride in methanol, may be used.

The demycarosyltylosin thioacetals represented by the general formula I in which X is a sulfur atom may be prepared by reacting tylosin or demycarosyltylosin with a thiol of formula $R^2SH$ or a disulfide $(R^2)_2S$. The thiol to be used may be a $C_{1-3}$ alkyl mercaptan or a thiophenol. Examples of $C_{1-3}$ alkylmercaptans are methylmercaptan, ethylmercaptan and propylmercaptan. The thiophenol may be thiophenol, a halogen-substituted phenylmercaptan such as chlorophenylmercaptan, or bromophenylmercaptan, a $C_{1-4}$ alkyl-substituted phenylmercaptan such as tolylmercaptan,

xylylmercaptan, ethylphenylmercaptan, propylphenylmercaptan or isopropylphenylmercaptan or a $C_{1-4}$ alkoxy-substituted phenylmercaptan such as methoxyphenylmercaptan, ethoxyphenylmercaptan or propoxyphenylmercaptan. The disulfide may be a $C_{1-3}$ alkyl disulfide or an aryl disulfide. As examples of the alkyl disulfide there may be mentioned methyl disulfide, ethyl disulfide or propyl disulfide. As the aryl disulfide there may be mentioned phenyl disulfide or a substituted phenyl disulfide such as methylphenyl disulfide or methoxyphenyl disulfide.

The thioacetalization may be carried out in the presence of a phosphine such as a tertiary phosphine using a suitable solvent. The phosphine to be used may be a tri $C_{1-4}$ alkylphosphine such as trimethylphosphine, triethylphosphine or tributylphosphine or a triarylphosphine such as triphenylphosphine. Tri-n-butylphosphine is the preferred reagent. The solvent to be used may be a chlorinated hydrocarbon such as chloroform, benzene or acetonitrile. The thioacetalization may be effected at temperatures from 0°C to the reflux temperature of the solvent, for periods ranging from about 15 minutes to 24 hours.

As indicated above, macrolide derivatives of formula IB:

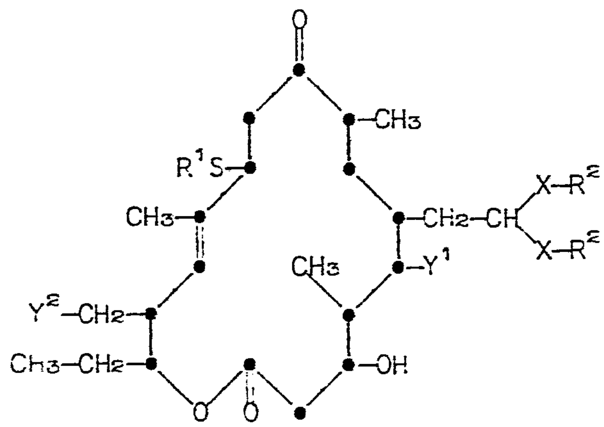

(IB)

may be prepared by reacting a tylosin acetal or thioacetal or demycarosyltylosin acetal or thioacetal of formula (IA) with a thiol of formula $R^1SH$. The thiol to be used may be a mercaptan or a thiophenol. The mercaptan may be a $C_{1-6}$ alkylmercaptan such as methylmercaptan, ethylmercaptan, propylmercaptan, isopropylmercaptan, butylmercaptan, tert.-butylmercaptan, pentylmercaptan or hexylmercaptan. The thiophenol may be phenylmercaptan, a halophenylmercaptan such as chlorophenylmercaptan or bromophenylmercaptan, an aminophenylmercaptan such as 4-aminophenylmercaptan a $C_{1-4}$ alkyl-substituted phenylmercaptan such as tolylmercaptan, xylylmercaptan, ethylphenylmercaptan, propylphenylmercaptan or a $C_{1-4}$ alkoxy-substituted phenylmercaptan such as methoxyphenylmercaptan or ethoxyphenylmercaptan, propoxyphenylmercaptan.

The thioetherification is preferably effected in the presence of a base using an appropriate solvent. The base to be used may be, for instance, an aliphatic tertiary amine such as triethylamine, a nitrogen-containing heterocyclic compound such as pyridine, an alkali metal alkoxide such as sodium methoxide, sodium ethoxide, sodium propoxide, potassium methoxide, potassium ethoxide, lithium methoxide or lithium ethoxide, or sodium hydride. A base such as pyridine may also be used as the solvent. As other solvents there may be mentioned chlorinated hydrocarbons such as chloroform, and alkanols such as methanol or ethanol. This thioetherification reaction is preferably conducted under a nitrogen atmosphere at the reflux temperature of the solvent to be used. The reaction time may range from 1 to 24 hours.

The macrolide derivatives of formula (IC):

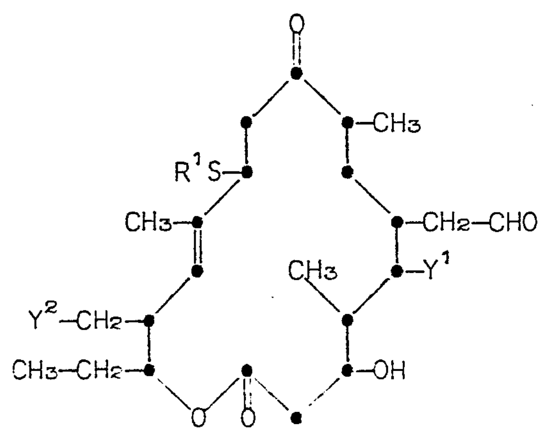

(IC)

may be prepared by cleaving the acetal or thioacetal group from C-20 by hydrolysis.

For compounds of formula (I) in which $Y^1$ is mycaminosyloxy, the hydrolysis may be accomplished using a mineral acid such as hydrochloric acid and using as solvent a water-miscible compound such as an alkanol, for example, methanol or ethanol, acetonitrile or an ether such as tetrahydrofuran. The reaction temperature may range from 0°C to the reflux temperature of the solvent, and the reaction time may range from about 30 minutes to 24 hours.

However, for the hydrolysis of compounds of formula (IC) in which $Y^1$ is mycarosyloxymycaminosyloxy, there is a risk that the mycarosyl group will be cleaved during the hydrolysis reaction. Accordingly, if it is desired to retain the mycarosyl group, mild hydrolysis conditions should be used. In this case, the hydrolysis may be appropriately carried out using an ether such as tetrahydrofuran or acetonitrile as the solvent at temperatures from about 0°C to 60°C for reaction periods ranging from about 30 minutes to 24 hours.

The diphenyl dithioacetal derivatives are preferably hydrolyzed with mercuric oxide and boron trifluoride etherate in aqueous tetrahydrofuran under a nitrogen atmosphere.

According to one aspect of the invention there is provided a process for preparing a compound of formula (I) which comprises:

(a) reacting tylosin or demycarosyltylosin with a compound of formula $R^2OH$, $R^2SH$ or $(R^2)_2S$ so as

to form an acetal or thioacetal of formula (I) in which Z represents:

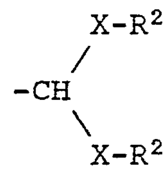

and A and B taken together represents a single chemical bond and/or;

(b) reacting a compound of formula (I) in which Z represents:

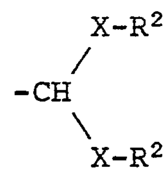

and A and B taken together represent a single bond, with a thiol of formula $R^1SH$ so as to form a compound of formula I in which A represents $SR^1$ and B is hydrogen; and/or

(c) hydrolysing a compound of formula (I) in which Z represents:

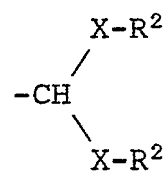

A represents $SR^1$ and B is hydrogen, so as to prepare a compound of formula (I) in which Z is -CHO, A is $SR^1$ and B is hydrogen; and

(d) optionally salifying any compound of formula I produced above so as to form the corresponding pharmacologically-acceptable salt.

Examples of pharmacologically-acceptable salts are acid-addition salts with an organic acid, e.g., tartaric acid, acetic acid, propionic acid, lactic acid, citric acid or succinic acid or an inorganic acid, e.g., hydrochloric acid, sulfuric acid or phosphoric acid. The pharmacologically acceptable salts may be prepared in conventional manner, for instance, by dissolving the compound of formula (I) in the form of a base and the relevant acid in a mixture of solvents, agitating the mixture and, if necessary, cooling to form the corresponding acid addition salt.

The compounds of formula I and their pharmacologically acceptable salts possess good antibacterial activity, particularly against gram-positive bacteria and, macrolide resistant strains. Accordingly, they can be used to treat or prevent bacterial infections in animals, including warm-blooded mammals.

Accordingly, in one aspect of the invention there is provided a veterinary or pharmaceutical formulation which comprises as an active ingredient a compound of formula (I), or a pharmacologically-acceptable salt thereof, together with one or more excipients or carriers therefor.

The antibacterial formulation according to the present invention may be administered in the form of a solid preparation such as a capsule, tablet or granule or in a liquid preparation such as a solution or suspension, orally or rectally, or parenterally

such as via an injection. The preparations described hereinabove may contain conventional excipients or carriers such as binders, suspending agents, emulsifying agents, buffers, etc.

Dosages will vary with the activity of the active ingredient and the therapeutic indication. However in general, typical dosages will range from 1 mg/kg to 100 mg/kg when administered as a pharmaceutical, and from 1 mg/kg to 1,000 mg/kg when administered as a veterinary.

The present invention will be described in more detail with reference to the following nonlimiting Examples.

## Example 1

Tylosin Dimethyl Acetal (Compound A)

To a solution of 1 gram of tylosin in 20 mℓ of methanol was added 0.34 mℓ of difluoroacetic acid, and the mixture was allowed to stand at room temperature for 2 days. After the completion of the reaction, the reaction mixture was neutralized with sodium hydrogen carbonate and then methanol was distilled off under reduced pressure to leave residues which in turn were mixed with 50 mℓ of water. The product was then extracted with three 50 mℓ portions of ethyl acetate and the resulting extract was dried over sodium sulfate. Concentration of the dried extract under reduced pressure yielded 800 mg of the crude title product as a

pale yellow solid, which was purified by silica gel column chromatography using a chloroform/methanol (10/1) mixture to yield 400 mg of pure tylosin dimethyl acetal (Compound A).

Proton NMR spectrum ($CDCl_3$)δ: 3.25, 3.30 (each 3H, s, C-20 $(OCH_3)_2$)

Mass spectrum: $M^+$ (m/z) 961

## Example 2

### Demycarosyltylosin Dimethyl Acetal (Compound B)

A solution of 10 grams of tylosin in 100 mℓ of a 0.5% hydrogen chloride-methanol mixture was allowed to react at room temperature for 1 hour. After the completion of the reaction, the reaction mixture was poured into a saturated sodium hydrogen carbonate aqueous solution and then extracted with chloroform. The resulting chloroform layer was dried over anhydrous sodium sulfate and concentrated under reduced pressure to dryness to give crude title product in solid form. This material was purified by silica gel column chromatography using a chloroform:methanol:concentrated ammonia (50:1:0.05) mixture to yield 8.5 grams (95% yield) of demycarosyltylosin dimethyl acetal (Compound B).

Mass spectrum (m/z): 817 ($M^+$), 468 (aglycone), 190, 174 (mycaminose)

Proton NMR spectrum δ(ppm):

1.69 (3H, 2, $H_{22}$), 2.49 (6H, s, $N(CH_3)_2$), 3.23, 3.28 (each 3H, s, acetal $OCH_3$), 3.45,

3.58 (each 3H, s, 2"-$OCH_3$, 3"-$OCH_3$), 4.30 (1H, d, J=7.5Hz, $H_{1'}$), 4.55 (1H, d, J=7.5Hz, $H_{1''}$), 4.96 (1H, bt, $J_{15,16}$=7.5Hz, $H_{15}$), 5.86 (1H, bd, J=11.0Hz, $H_{13}$), 6.25 (1H, d, J=15.0Hz, $H_{10}$), 7.28 (1H, d, J=15.0Hz, $H_{11}$)

## Example 3

### Tylosin Diphenyl Thioacetal (Compound C)

To a solution of 10 grams of tylosin and 3.6 grams of phenyl disulfide in 80 mℓ of chloroform were added 4.5 mℓ of tri-n-butylphosphine under a nitrogen atmosphere. The mixture was allowed to react at room temperature for 2 hours. After completion of the reaction, the resulting reaction mixture was concentrated under reduced pressure and purified by means of silica gel column chromatography using a chloroform/methanol/conc. ammonia (50/1/0.05) eluent to yield 7.9 grams (65%) of tylosin diphenyl thioacetal (Compound C).

Mass spectrum (m/z): 1117 ($M^+$), 608 (aglycone), 318 (mycarosylmycaminose), 191 (mycinose)

Proton NMR spectrum, δ(ppm):

1.77 (3H, s, $H_{22}$), 2.57 (6H, s, $N(CH_3)_2$), 3.45, 3.59 (each 3H, s, 2'"-$OCH_3$, 3'"-$OCH_3$), 4.16 (1H, d, J=7.5Hz, $H_{1'}$), 4.54 (1H, d, J=8.0Hz, $H_{1'''}$), 5.0(b, $H_{15}$), 5.06 (b, $H_{1'''}$), 5.83 (1H, d, J=9.5Hz, $H_{13}$), 6.23 (1H, d, J=13.5Hz, $H_{10}$), 7.1 - 7.6 (m, $H_{11}$, aromatic ring proton)

## Example 4

### 10,11-Dihydro-11-phenylthiotylosin Diphenyl thioacetal (Compound D)

A solution of 1.0 gram of tylosin diphenyl thioacetal (Compound C) and 1.0 mℓ of thiophenol in 20 mℓ of triethylamine was refluxed for 4 hours under a nitrogen atmosphere. After completion of the reaction, the resulting reaction mixture was concentrated under reduced pressure and purified by means of silica gel column chromatography using a chloroform/methanol/ conc. ammonia (40/1/0.05) mixture to yield 438 mg (40%) of 10,11-dihydro-11-phenylthiotylosin diphenylthio acetal (Compound D).

Mass spectrum (m/z): 718, 702(aglycone), 191 (mycinose), 174 (mycaminose)

Proton NMR spectrum, δ (ppm):

1.63 (3H, s, $H_{22}$), 2.46 (6H, s, $N(CH_3)_2$), 3.43, 3.56 (each 3H, s, 2'"-$OCH_3$, 3'"-$OCH_3$), 4.35 (1H, d, J=7.5Hz, $H_{1'''}$), 4.67 (1H, bd, J=9.0Hz, $H_{13}$), 4.8 (b, $H_{15}$), 5.00 (bd, 1H, $H_{1''}$), 7.2 (m, aromatic ring proton)

## Example 5

### 11-(p-Aminophenylthio)-10,11-dihydrotylosin Diphenylthioacetal (Compound E)

A solution of 100 mg of tylosin diphenylthio acetal (Compound C) and 70 mg of p-aminothiophenol in 2 mℓ of triethylamine was refluxed for 5 hours under

a nitrogen atmosphere. After the completion of the reaction, the resulting reaction mixture was concentrated under reduced pressure and purified by means of silica gel column chromatography using a chloroform/methanol/conc. ammonia (15/1/0.05) mixture to yield 64 mg (58%) of 11-(p-aminophenylthio)-10,11-dihydrotylosin diphenylthioacetal (Compound E).

Mass spectrum (m/z): 717 (aglycone), 191 (mycinose), 174 (mycaminose)

Proton NMR spectrum, δ (ppm):

1.66 (3H, s, $H_{22}$), 2.46 (6H, s, $N(CH_3)_2$), 3.48, 3.60 (each 3H, s, 2'''-$OCH_3$, 3'''-$OCH_3$), 4.44 (1H, d, J=8.0Hz, $H_{1''}$), 4.60 (1H, bd, J=9.0Hz, $H_{13}$), 4.9 (b, $H_{15}$), 5.03 (1H, d, J=3.0Hz, $H_{1'''}$), 6.42, 6.95 (each 2H, d, J=8.0Hz, $-SC_6\underline{H}_4NH_2$), 7.3 (m, aromatic ring proton)

## Example 6

### 11-(p-Methylphenylthio)-10,11-dihydrotylosin Diphenylthioacetal (Compound F)

A solution of 2.0 grams of tylosin diphenylthio acetal (Compound C) and 2.3 grams of p-thiocresol in 40 mℓ of triethylamine was reacted under reflux for three hours. After the completion of the reaction, the resulting reaction mixture was concentrated under reduced pressure and then purified by means of silica gel column chromatography using a chloroform/methanol/conc. ammonia (50/1/0.05) mixture to give 860 mg (39%)

of 11-(p-methylphenylthio)-10,11-dihydrotylosin diphenylthio acetal (Compound F).

Mass spectrum (m/z): 1241($M^+$), 1096($M^+$-mycarose), 191, 175 (mycinose), 161, 145 (mycarose), 174 (mycaminose)

Proton NMR spectrum, δ (ppm):

1.63 (3H, s, $H_{22}$), 2.25 (3H, s, $-SC_6H_4C\underline{H}_3$), 2.45 (6H, s, $N(CH_3)_2$), 3.44, 3.57 (each 3H, s, 2'"-$OCH_3$, 3'"-$OCH_3$), 4.10 (1H, d, J=7.0Hz, $H_{1'}$), 4.36 (1H, d, J=8.0Hz, $H_{1'''}$), 4.63 (1H, bd, J=9.0Hz, $H_{13}$), 4.8 (b, $H_{15}$), 5.00 (1H, d, J=3.0Hz, $H_{1''}$), 6.9 - 7.5 (m, aromatic ring proton)

## Example 7

### 11-(p-Chlorophenylthio)-10,11-dihydrotylosin Diphenylthioacetal (Compound G)

A solution of 1.0 gram of tylosin diphenylthioacetal (Compound C) and 1.3 grams of p-chlorothiophenol in 20 mℓ of triethylamine was reacted under reflux for 24 hours under a nitrogen atmosphere. After the completion of the reaction, the resulting reaction mixture was concentrated under reduced pressure and purified by means of silica gel column chromatography using a chloroform/methanol/conc. ammonia mixture (50/1/0.05) to yield 418 mg (37%) of 11-(p-chlorophenylthio)-10,11-dihydrotylosin diphenylthioacetal (Compound G).

Mass spectrum (m/z): 927 (aglycone + mycinose), 191, 175 (mycinose), 174 (mycaminose), 161 (mycarose)

Proton NMR spectrum, δ(ppm):

1.63 (3H, s, $H_{22}$), 2.47 (6H, s, $N(CH_3)_2$), 3.46, 3.60 (each 3H, s, 2'"-$OCH_3$, 3'"-$OCH_3$), 4.10 (1H, d, J=7.0Hz, $H_{1'}$), 4.40 (1H, d, J=7.5Hz, $H_{1'''}$), 4.69 (1H, bd, J=9.0Hz, $H_{13}$), 4.9 (b, $H_{15}$), 5.05 (1H, bd, J=3.0Hz, $H_{1''}$), 7.0 - 7.4 (m, aromatic ring proton)

## Example 8

### 11-(p-Methoxyphenylthio)-10,11-dihydrotylosin Diphenylthioacetal (Compound H)

A solution of 1.0 gram of tylosin diphenylthioacetal (Compound C) and 1.5 mℓ of p-methoxythiophenol in 20 mℓ of triethylamine was reacted under reflux for 4 hours under a nitrogen atmosphere. After completion of the reaction, the resulting reaction mixture was concentrated under reduced pressure and purified by means of silica gel column chromatography using a chloroform/methanol/conc. ammonia mixture (40/1/0.05) to yield 416 mg (37%) of 11-(p-methoxyphenylthio)-10,11-dihydrotylosin diphenylthioacetal (Compound H).

Mass spectrum (m/z): 764 (aglycone), 191, 175 (mycinose), 174 (mycaminose), 161, 145 (mycarose)

Proton NMR spectrum, δ (ppm):

1.64 (3H, s, $H_{22}$), 2.45 (6H, s, $N(CH_3)_2$), 3.44 3.56 (each 3H, s, 2'"-$OCH_3$, 3'"-$OCH_3$), 3.68 (3H, s, -$SC_6H_4OC\underline{H}_3$), 4.09 (1H, d, J= 7.0Hz, $H_{1'}$), 4.38 (1H, d, J=7.5Hz, $H_{1'''}$), 4.56 (1H, bd, J=9.0Hz, $H_{13}$), 4.8 (b, $H_{15}$), 5.00 (1H, d, J=3.0Hz, $H_{1''}$), 6.5-7.4 (m, aromatic ring proton)

## Example 9

### 11-Ethylthio-10,11-dihydrotylosin Diphenylthioacetal (Compound I)

A solution of 200 mg of tylosin diphenylthioacetal (Compound C) and 0.2 mℓ of ethanethiol in 4 mℓ of triethylamine was reacted under reflux for 4.5 hours under a nitrogen atmosphere. After completion of the reaction, the resulting reaction mixture was concentrated under reduced pressure and then purified by means of silica gel column chromatography using a chloroform/methanol/conc. ammonia mixture (15/1/0.05) to yield 150 mg (71%) of 11-ethylthio-10,11-dihydrotylosin diphenylthioacetal (Compound I).

Mass spectrum (m/z): 845 (aglycone + mycinose), 654 (aglycone), 191 (mycinose), 145 (mycarose)

Proton NMR spectrum, δ(ppm):

1.63 (3H, s, $H_{22}$), 2.49 (6H, s, $N(CH_3)_2$), 3.48, 3.57 (each 3H, s, 2'"-$OCH_3$), 4.16 (1H, d, J=7.0Hz, $H_{1'}$), 4.48 (1H, d, J=7.5Hz, $H_{1'''}$), 5.0 (b, $H_{13}$, $H_{15}$, $H_{1''}$), 7.3 (m, aromatic ring proton)

## Example 10

### 10,11-Dihydro-11-ethylthiodemycarosyltylosin Dimethylacetal (Compound J)

A solution of 200 mg of demycarosyltylosin dimethylacetal (Compound B) and 0.2 mℓ of ethanethiol in 4 mℓ of triethylamine was reacted under reflux for 5 hours under a nitrogen atmosphere. After completion of the reaction, the resulting reaction mixture was concentrated under reduced pressure and then purified by means of silica gel column chromatography using a chloroform/methanol/conc. ammonia mixture (20/1/0.05) to yield 182 mg (85%) of 10,11-dihydro-11-ethylthiodemycarosyltylosin dimethylacetal (Compound J).

Mass spectrum (m/z): 879 ($M^+$), 688 ($M^+$-mycinose), 174 (mycaminose)

Proton NMR spectrum, δ(ppm):

1.67 (3H, s, $H_{22}$), 2.50 (6H, s, $N(CH_3)_2$), 3.27 (6H, s, acetal-$OCH_3$), 3.50, 3.59 (each 3H, s, 2"-$OCH_3$, 3"-$OCH_3$), 4.33 (1H, d, J=7.0Hz, $H_{1'}$), 4.53 (1H, d, J=7.5Hz, $H_{1''}$), 5.1 (6, $H_{15}$), 5.20 (1H, bd, J=9.5Hz, $H_{13}$)

## Example 11

### 10,11-Dihydro-11-(2-hydroxyethylthio)demycarosyltylosin Dimethylacetal (Compound K)

A solution of 100 mg of demycarosyltylosin dimethylacetal and 0.1 mℓ of 2-mercaptoethanol in 2 mℓ of triethylamine was reacted under reflux for 6.5 hours

under a nitrogen atmosphere. After the reaction was over the resulting reaction mixture was concentrated under reduced pressure and then purified by means of silica gel column chromatography using a chloroform/methanol/conc. ammonia mixture (20/1/0.05) to yield 40 mg (37%) of 10,11-dihydro-11-(2-hydroxyethylthio)-demycarosyltylosin dimethylacetal (Compound K).

Mass spectrum (m/z): 530 (aglycone), 174 (mycaminose)

Proton NMR spectrum, δ(ppm):

1.83 (3H, s, $H_{22}$), 2.59 (6H, s, $N(CH_3)_2$), 3.25 (6H, s, acetal $OCH_3$), 3.47, 3.56 (each 3H, s, 2"-$OCH_3$, 3"-$OCH_3$), 4.41 (1H, d, J=7.5Hz, $H_{1'}$), 4.50 (1H, d, J=7.5Hz, $H_{1''}$), 5.0(b, $H_{15}$), 5.10 (1H, bd, J=8.5Hz, $H_{13}$)

## Example 12

### 10,11-Dihydro-11-phenylthiodemycarosyltylosin Dimethylacetal (Compound L)

A solution of 100 mg of demycarosyltylosin dimethylacetal (Compound B) and 0.15 mℓ of thiophenol in 2 mℓ of triethylamine was reacted under reflux for 7 hours under a nitrogen atmosphere. After the reaction was over, the resulting reaction mixture was concentrated under reduced pressure and then purified by means of silica gel column chromatography using a chloroform/methanol/conc. ammonia mixture (15/1/0.05) to yield 42 mg (37%) of 10,11-dihydro-11-phenylthiodemycarosyltylosin dimethylacetal (Compound L).

Mass spectrum (m/z): 927($M^+$), 753 ($M^+$-mycaminose), 190, 174 (mycaminose)

Proton NMR spectrum, δ(ppm):

1.63 (3H, s, $H_{22}$), 2.49 (6H, s, $N(CH_3)_2$), 3.23 (6H, s, acetal-$OCH_3$), 3.44, 3.56 (each 3H, s, 2"-$OCH_3$, 3"-$OCH_3$), 4.31 (1H, d, J=7.0Hz, $H_{1'}$), 4.40 (1H, d, J=8.0Hz, $H_{1''}$), 4.9 (b, $H_{13}$, $H_{15}$, $H_{1''}$), 7.3 (m, aromatic ring proton)

## Example 13

10,11-Dihydro-11-(p-aminophenylthio)demycarosyltylosin Dimethylacetal (Compound M)

A solution of 100 mg of demycarosyltylosin dimethylacetal (Compound B) and 150 mg of p-aminothiophenol in 2 mℓ of triethylamine was reacted under reflux for 4.5 hours under a nitrogen atmosphere. After the reaction was over, the resulting reaction mixture was concentrated under reduced pressure and then purified by means of silica gel column chromatography using a chloroform/methanol/conc. ammonia mixture (15/1/0.05) to yield 41 mg (36%) of 10,11-dihydro-11-(p-aminophenylthio)demycarosyltylosin dimethylacetal (Compound M).

Mass spectrum (m/z): 768($M^+$-mycaminose), 190 (mycaminose), 175 (mycinose)

Proton NMR spectrum, δ(ppm):

1.69 (3H, s, $H_{22}$), 2.52 (6H, s, $N(CH_3)_2$), 3.26 (6H, s, acetal-$OCH_3$), 3.48, 3.59 (each 3H, s, 2"-$OCH_3$, 3"-$OCH_3$), 4.31 (1H, d,

$J=8.0Hz$, $H_{1'}$), 4.45 (1H, d, $J=8.0Hz$, $H_{1''}$), 4.77 (1H, bd, $J=11.0Hz$, $H_{13}$), 4.92 (1H, bt, $J_{15,16}=8.0Hz$, $H_{15}$), 6.56, 7.16 (each 2H, d, $J=8.0Hz$, aromatic ring proton)

## Example 14

### 10,11-Dihydro-11-ethylthiodemycarosyltylosin (Compound N)

A solution of 120 mg of 10,11-dihydro-11-ethylthiodemycarosyltylosin dimethylacetal (Compound J) in 1.5 mℓ of 0.1N HCℓ-acetonitrile (2.5:1) was reacted at room temperature for 1 hour. After the reaction was over, the resulting reaction mixture was poured into a saturated sodium hydrogen carbonate aqueous solution and then extracted with chloroform. The chloroform layer was then dried over anhydrous sodium sulfate and concentrated to dryness under reduced pressure to give crude product purified by means of silica gel thin layer chromatography using a chloroform/methanol/conc. ammonia mixture (10/1/0.05) to yield 105 mg (92%) of 10,11-dihydro-11-ethylthiodemycarosyltylosin (Compound N).

$[\alpha]_D^{27}$ : -14.9 (c 1, methanol)

uv: $\lambda_{max}^{MeOH}nm(\varepsilon)$: 282 (2,7000)

Mass spectrum (m/z): 833 ($M^+$), 642 ($M^+$-mycinose), 174 (mycaminose)

Proton NMR spectrum, δ(ppm):

1.67 (3H, s, $H_{22}$), 2.46 (6H, s, $N(CH_3)_2$), 3.46, 3.55 (each 3H, s, 2"-$OCH_3$, 3"-$OCH_3$), 4.26 (1H, d, J=8.0Hz, $H_{1'}$), 4.48 (1H, d, J=8.0Hz, $H_{1''}$), 5.0 (b, $H_{15}$), 5.17 (1H, bd, J=9.5Hz, $H_{13}$), 9.66 (1H, s, $H_{20}$)

## Example 15

### 10,11-Dihydro-11-(2-hydroxyethylthio)-demycarosyltylosin (Compound O)

A solution of 30 mg of 10,11-dihydro-11-(2-hydroxyethylthio)demycarosyltylosin dimethylacetal (Compound K) in 1 mℓ of 0.1N HCℓ-acetonitrile (2.5:1) was reacted at room temperature for 1 hour. After the reaction was over, the resulting reaction mixture was treated in substantially the same manner as in Example 14 to yield 23 mg (81%) of 10,11-dihydro-11-(2-hydroxyethylthio)demycarosyltylosin (Compound O).

$[\alpha]_D^{27}$: -34.4° (c 0.5, methanol)

uv : $\lambda_{max}^{MeOH}$nm(ε) : 285 (2,100)

Mass spectrum (m/z): 468 (aglycone), 174 (mycaminose)

Proton NMR spectrum, δ(ppm):

1.81 (3H, s, $H_{22}$), 2.47 (6H, s, $N(CH_3)_2$), 3.46, 3.56 (each 3H, s, 2"-$OCH_3$, 3"-$OCH_3$), 4.33 (1H, s, J=7.5Hz, $H_{1'}$), 4.50 (1H, s, J=7.5Hz, $H_{1''}$), 5.08 (1H, bd, J=9.0Hz, $H_{13}$), 5.1 (b, $H_{15}$), 9.67 (1H, s, $H_{20}$)

Example 16

10,11-Dihydro-11-(p-aminophenylthio)-demycarosyltylosin (Compound P)

A solution of 40 mg of 10,11-dihydro-11-(p-aminophenylthio)demycarosyltylosin dimethylacetal (Compound M) in 1 mℓ of 0.1N HCℓ-acetonitrile(2.5:1) was reacted at room temperature for 1 hour. After the reaction was over, the resulting reaction mixture was treated in substantially the same manner as in Example 14 to yield 32 mg (84%) of 10,11-dihydro-11-(p-aminophenylthio)demycarosyltylosin (Compound P).

$[\alpha]_D^{27}$: -18.3°(c 1, methanol)

uv : $\lambda_{max}^{MeOH}$ nm(ε): 271 (13,000)

Mass spectrum (m/z): 706($M^+$-mycaminose), 531 (aglycone), 191, 175 (mycinose), 190, 174 (mycaminose)

Proton NMR spectrum, δ(ppm):

1.69 (3H, s, $H_{22}$), 2.52 (6H, s, $N(CH_3)_2$), 3.48, 3.59 (each 3H, s, 2"-$OCH_3$, 3"-$OCH_3$), 4.31 (1H, d, J=8.0Hz, $H_{1'}$), 4.45 (1H, d, J=8.0Hz, $H_{1''}$), 4.77 (1H, bd, J=10.5Hz, $H_{13}$), 4.92 (1H, bt, $J_{15,16}$=8.0Hz, $H_{15}$), 6.56, 7.16 (each 2H, d, J=8.0Hz, aromatic ring proton), 9.67(1H, s, $H_{20}$)

## Example 17

### 10,11-Dihydro-11-phenylthiodemycarosyltylosin (Compound Q)

A solution of 40 mg of 10,11-dihydro-11-phenylthiodemycarosyltylosin dimethylacetal (Compound L) in 1 mℓ of 0.1N HCℓ-acetonitrile was reacted at room temperature for 1 hour. After the reaction was over, the resulting reaction mixture was treated in substantially the same manner as in Example 14 to yield 25 mg (66%) of 10,11-dihydro-11-phenylthiodemycarosyltylosin (Compound Q).

$[\alpha]_D^{27}$: -11.4° (c 1, methanol)

uv : $\lambda_{max}^{MeOH}$nm($\varepsilon$): 264 (4,700)

Mass spectrum (m/z): 881($M^+$), 190, 174 (mycaminose)

Proton NMR spectrum, δ(ppm):
1.66 (3H, s, $H_{22}$), 2.47 (6H, s, $N(CH_3)_2$), 3.45, 3.54 (each 3H, s, 2"-$OCH_3$, 3"-$OCH_3$), 4.25 (1H, d, J=8.0Hz, $H_{1'}$), 4.37 (1H, d, J=7.5Hz, $H_{1''}$), 4.91 (1H, bd, J=9.0Hz, $H_{13}$), 4.8-5.0(b, $H_{15}$), 7.2-7.3 (m, aromatic ring proton), 9.66 (1H, s, $H_{20}$)

## Example 18

10,11-Dihydro-11-ethylthiotylosin (Compound R)

A solution of 150 mg of 10,11-dihydro-11-ethylthiotylosin diphenylthioacetal (Compound I), 28 mg of mercuric oxide and 0.02 mℓ of $BF_3$ etherate in 1.5 mℓ of 15% aqueous tetrahydrofuran was reacted at room temperature for 1 hour under a nitrogen atmosphere. After the reaction was over, the resulting reaction mixture was poured into a saturated sodium hydrogen carbonate aqueous solution and extracted with diethylether. The resulting ether layer was dried over anhydrous sodium sulfate and concentrated under reduced pressure to dryness to give crude product which in turn was purified by means of silica gel thin layer chromatography using a chloroform/methanol/conc. ammonia mixture (12/1/0.05) to yield 37 mg (30%) of 10,11-dihydro-11-ethylthiotylosin (Compound R).

$[\alpha]_D^{27}$: -50.0° (c 1, methanol)

uv: $\lambda_{max}^{MeOH}$ nm(ε): 285 (7,100)

Mass spectrum (m/z): 641 (aglycone + mycaminose), 484, 452 (aglycone), 175 (mycinose), 174 (mycaminose)

Proton NMR spectrum, δ(ppm):

1.64 (3H, s, $H_{22}$), 2.45 (6H, s, $N(CH_3)_2$), 3.47, 3.56 (each 3H, s, 2'"-$OCH_3$, 3'"-$OCH_3$), 4.50 (1H, d, J=8.0Hz, $H_{1'}$), 4.52 (1H, d, J=8.0Hz, $H_{1'''}$), 5.0 (3H, b, $H_{13}$, $H_{15}$, $H_{1''}$), 9.66 (1H, s, $H_{20}$)

Example 19

10,11-Dihydro-11-phenylthiotylosin (Compound S)

A solution of 400 mg of 10,11-dihydro-11-phenylthiotylosin diphenylthioacetal (Compound D), 310 mg of mercuric oxide and 0.2 mℓ of $BF_3$ etherate in 4 mℓ of 15% aqueous tetrahydrofuran was reacted at room temperature for 1 hour under a nitrogen atmosphere. After the reaction was over, the resultant reaction mixture was treated in substantially the same manner as in Example 18 to yield 185 mg (55%) of 10,11-dihydro-11-phenylthiotylosin (Compound S).

$[\alpha]_D^{27}$: -40.8° (c 1, methanol)

uv: $\lambda_{max}^{MeOH}$nm(ε): 262 (5,000)

Mass spectrum (m/z): 864($M^+$-mycarose), 191 (mycinose), 174 (mycaminose)

Proton NMR spectrum, δ(ppm):

1.63 (3H, s, $H_{22}$), 2.47 (6H, s, $N(CH_3)_2$), 3.41, 3.54 (each 3H, s, 2'"-$OCH_3$, 3'"-$OCH_3$), 4.18 (1H, d, J=7.5Hz, $H_{1'}$), 4.36 (1H, d, J=7.5Hz, $H_{1'''}$), 4.84 (1H, d, J=9.5Hz, $H_{13}$), 4.90 (b, $H_{15}$), 4.99 (1H, bd, J=3.0Hz, $H_{1''}$), 7.2 (m, aromatic ring proton), 9.75 (1H, s, $H_{20}$)

## Example 20

### 10,11-Dihydro-11-(p-chlorophenylthio)tylosin (Compound T)

A solution of 300 mg of 10,11-dihydro-11-(p-chlorophenylthio)tylosin diphenylthioacetal (Compound G), 30 mg of mercuric oxide and 0.02 mℓ of $BF_3$ etherate in 3 mℓ of 15% aqueous tetrahydrofuran was reacted at room temperature for 1 hour under a nitrogen atmosphere. After the reaction was over, the resultant reaction mixture was treated in substantially the same manner as in Example 18 to yield 110 mg (44%) of 10,11-dihydro-11-(p-chlorophenylthio)tylosin (Compound T).

$[\alpha]_D^{27}$: -53.6° (c 1, methanol)

uv: $\lambda_{max}^{MeOH}$nm(ε): 266 (8,500)

Mass spectrum (m/z): 898 ($M^+$-mycarose), 725 (aglycone + mycinose), 566 (aglycone), 191, 175 (mycinose), 174 (mycaminose)

Proton NMR spectrum, δ(ppm):
1.66 (3H, s, $H_{22}$), 2.52 (6H, s, $N(CH_3)_2$), 3.46, 3.59 (each 3H, s, 2'"-$OCH_3$, 3'"-$OCH_3$), 4.23 (1H, d, J=7.5Hz, $H_{1'}$), 4.43 (1H, d, J=7.5Hz, $H_{1''}$), 4.62 (1H, bd, J=9.0Hz, $H_{22}$), 4.9 (b, $H_{15}$), 5.04 (1H, d, J=3.0Hz, $H_{1''}$), 7.26 (s, aromatic ring proton)., 9.67 (1H, s, $H_{20}$)

## Example 21

### 10,11-Dihydro-11-(p-aminophenylthio)tylosin (Compound U)

A solution of 100 mg of 10,11-dihydro-11-(p-aminophenylthio)tylosin diphenylthioacetal (Compound E), 110 mg of mercuric oxide and 0.07 mℓ of $BF_3$ etherate in 1 mℓ of 15% aqueous tetrahydrofuran was reacted at room temperature for 1 hour under a nitrogen atmosphere. After the reaction was over, the resultant reaction mixture was treated in substantially the same manner as in Example 18 to yield 15 mg (18%) of 10,11-dihydro-11-(p-aminophenylthio)tylosin (Compound U).

$[\alpha]_D^{27}$: -59.6° (c 0.1, methanol)

uv: $\lambda_{max}^{MeOH}$nm(ε): 274 (16,000)

Mass spectrum (m/z): 531 (aglycone), 191 (mycinose), 174 (mycaminose)

Proton NMR spectrum, δ(ppm):

1.75 (3H, s, $H_{22}$), 2.53 (6H, s, $N(CH_3)_2$), 3.51, 3.60 (each 3H, s, 2'"-$OCH_3$, 3'"-$OCH_3$), 4.07 (1H, d, J=8.0Hz, $H_{1'}$), 4.54 (1H, d, J=8.0Hz, $H_{1'''}$), 5.1 (3H, b, $H_{1''}$, $H_{15}$, $H_{13}$), 6.55, 7.13 (each 2H, d, aromatic ring proton), 9.70 (1H, s, $H_{20}$)

Example 22

10,11-Dihydro-11-(p-methylphenylthio)tylosin (Compound V)

A solution of 200 mg of 10,11-dihydro-11-(p-methylphenylthio)tylosin diphenylthioacetal (Compound F), 140 mg of mercuric oxide and 0.1 mℓ of $BF_3$ etherate in 2 mℓ of 15% aqueous tetrahydrofuran was reacted at room temperature for 1 hour under a nitrogen atmosphere. After the reaction was over, the resultant reaction mixture was treated in substantially the same manner as in Example 18 to yield 47 mg (28%) of 10,11-dihydro-11-(p-methylphenylthio)tylosin (Compound V).

$[\alpha]_D^{27}$: -46.0° (c 1, methanol)

uv: $\lambda_{max}^{MeOH}$ nm(ε): 263 (6,700)

Mass spectrum (m/z): 894($M^+$-mycarose), 706 (aglycone + mycinose), 318 (mycarosyl mycamincse), 174 (mycaminose), 145 (mycarose)

Proton NMR spectrum, δ(ppm):

1.66 (3H, s, $H_{22}$), 2.30 (3H, s, $-SC_6H_4C\underline{H}_3$), 2.49 (6H, s, $N(CH_3)_2$), 3.46, 3.57 (each 3H, s, 2'"-$OCH_3$, 3'"-$OCH_3$), 4.43 (1H, d, J=8.0Hz, $H_{1''}$), 4.9 (2H, b, $H_{13}$, $H_{15}$), 5.02 (1H, d, J=3.0Hz, $H_{1''}$), 7.03, 7.23 (each 2H, d, J=8.0Hz, aromatic ring proton), 9.65 (1H, s, $H_{20}$)

Example 23

10,11-Dihydro-11-(p-methoxyphenylthio)tylosin (Ccmpound W)

A solution of 400 mg of 10,11-dihydro-11-(p-methoxyphenylthio)tylosin diphenylthioacetal (Compound H), 70 mg of mercuric oxide and 0.05 mℓ of $BF_3$ etherate in 4 mℓ of 15% aqueous tetrahydrofuran was reacted at room temperature for 1 hour under a nitrogen atmosphere. After the reaction was over, the resultant reaction mixture was treated in substantially the same manner as in Example 18 to yield 170 mg (51%) of 10,11-dihydro-11-(p-methoxyphenylthio)tylosin (Compound W).

$[\alpha]_D^{27}$: -49.7° (c 1, methanol)

uv : $\lambda_{max}^{MeOH}$ nm(ε): 262 (5,400), 231 (10,200)

Mass spectrum (m/z): 562, 530 (aglycone), 318 (mycarosylmycaminose), 174 (mycaminose)

Proton NMR spectrum, δ(ppm):

1.69 (3H, s, $H_{22}$), 2.57 (6H, s, $N(CH_3)_2$), 3.47, 3.57 (each 3H, s, 2""-$OCH_3$, 3'"-$OCH_3$), 3.76 (3H, s, $-SC_6H_4OC\underline{H}_3$), 4.26 (1H, d, J=7.0Hz, $H_{1'}$), 4.43 (1H, d, J=7.5Hz, $H_{1''''}$), 4.78 (1H, bd, J=9.0Hz, $H_{13}$), 4.9 (b, $H_{15}$), 5.05 (1H, d, J=3.CHz, $H_{1''}$), 6.78 (each 2H, d, J=9.0Hz, aromatic ring proton), 9.66 (1H, s, $H_{20}$)

The compounds of formula (I) show antibacterial activity against both gram-positive and gram-negative bacteria. The following Tables illustrate minimum inhibitory concentrations for a representative sample of the compounds of the invention against gram-positive and gram-negative bacteria as well as bacteria resistant to antibiotics. In the Tables abbreviations with the symbol r attached designate resistant bacterial strains.

EM = erythromycin
TC = tetracycline
PC = penicillin
CP = chloramphenicol
MC = macrolide
KM = kanamycin
SM = streptomycin

Table 1

Compounds (MIC μg/ml)

| Bacteria | A | B | C | N | O | P | Q | R | S | T | U | V | W |
|---|---|---|---|---|---|---|---|---|---|---|---|---|---|
| Staphylococcus aureus ATCC 6538P | 3.12 | 1.56 | 12.5 | 1.56 | 12.5 | 3.12 | 0.78 | 1.56 | 1.56 | 3.12 | 6.25 | 3.12 | 3.12 |
| Staphylococcus aureus KB 199(EM$^r$) | >100 | >100 | >50 | >100 | >100 | >100 | >100 | >100 | 50 | 25 | >100 | 25 | 50 |
| Bacillus subtilis PCI 219 | 0.4 | 3.12 | 0.78 | 0.78 | 12.5 | 3.12 | 0.78 | 0.78 | 0.4 | 0.4 | 0.4 | 0.4 | 0.4 |
| Bacillus cereus IFO 3001 | 0.78 | 0.78 | 0.78 | 0.78 | 3.12 | 0.78 | 0.2 | 0.78 | 0.4 | 0.4 | 1.56 | 0.4 | 0.4 |
| Micrococcus luteus ATCC 9341 | 0.39 | 0.2 | 0.2 | 0.2 | 0.4 | 0.2 | <0.1 | <0.1 | <0.1 | <0.1 | 0.78 | <0.1 | <0.1 |
| Mycobacterium smegmatis ATCC 607 | 100 | 100 | 50 | >100 | >100 | >100 | >100 | 100 | 50 | 50 | >100 | 50 | >100 |
| Escherichia coli NIHJ | >100 | >100 | >100 | >100 | >100 | >100 | 100 | 100 | 100 | 100 | >100 | 100 | 100 |
| Klebsiella pneumoniae ATCC 10031 | >100 | 100 | >100 | 50 | >100 | 100 | 100 | 100 | 100 | 100 | >100 | 100 | 100 |
| Klebsiella pneumoniae PCI 602 | >100 | 50 | >100 | >100 | >100 | >100 | 100 | 100 | 100 | 100 | >100 | 100 | 100 |
| Salmonella typhirium KB 20 | >100 | >100 | >100 | >100 | >100 | >100 | 100 | >100 | 100 | 100 | >100 | 100 | 100 |
| Proteus vulgaris IFO 3167 | >100 | >100 | >100 | >100 | >100 | >100 | 50 | >100 | 100 | 50 | >100 | 50 | 50 |
| Pseudomonas aeruginosa P-3 | - | - | - | >100 | >100 | >100 | >100 | >100 | >100 | >100 | >100 | >100 | >100 |

Table 2

| Resistant Bacteria | Compounds S | T | U | W |
|---|---|---|---|---|
| Staphylococcus aureus KB 191 (MC, $TC^r$) | 50 | 25 | 25 | 50 |
| Staphylococcus aureus KB 219 (MC, TC, PC, $CP^r$) | 100 | 50 | 50 | 100 |
| Staphylococcus aureus KB 221 (MC, TC, CP, $KM^r$) | 50 | - | - | - |
| Staphylococcus aureus KB 224 (MC, TC, KM, $SM^r$) | 50 | 25 | 25 | 50 |

CLAIMS

1. A compound of formula (I):

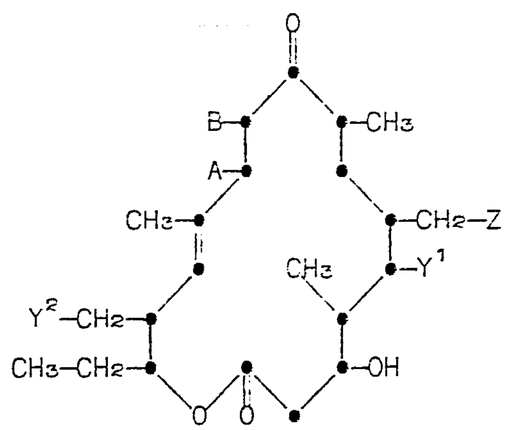

(I)

wherein A represents a group of formula $-SR^1$ where $R^1$ is a $C_{1-6}$ alkyl or $C_{2-6}$ hydroxyalkyl group or a monovalent aryl group when B represents a hydrogen atom;

or A and B taken together represent a single chemical bond;

wherein $Y^1$ represents a group of formula:

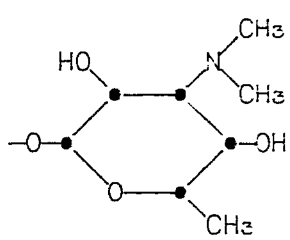

or

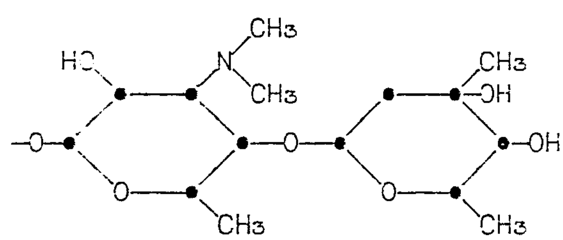

wherein $Y^2$ represents a group of formula:

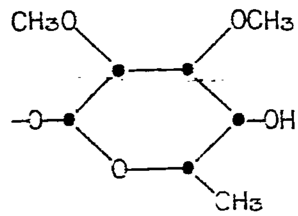

and Z represents CHO or a group of formula

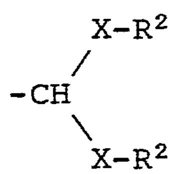

where X represents an oxygen or sulfur atom and $R^2$ is $C_{1-3}$ alkyl or aryl;

provided that when A and B taken together represent a single chemical bond then Z cannot be -CHO and X cannot be oxygen;

or a pharmacologically-acceptable salt thereof.

2. A compound of formula (I) in which Z is -CHO and A is $-SR^1$ where $R^1$ is an optionally substituted phenyl group.

3. 10,11-Dihydro-11-phenylthiotylosin.

4. 10,11-Dihydro-11-(p-chlorophenylthio)-tylosin.

5. 10,11-Dihydro-11-(p-aminophenylthio)-tylosin.

6. 10,11-Dihydro-11-(p-methylphenylthio)-tylosin.

7. A process for preparing a compound of formula (I), or a pharmacologically-acceptable salt thereof, as claimed in any one of claims 1 to 6 which comprises:

(a) reacting tylosin or demycarosyltylosin with a compound of formula $R^2OH$, $R^2SH$ or $(R^2)_2S$ so as to form an acetal or thioacetal of formula (I) in which Z represents:

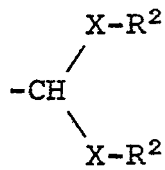

and A and B taken together represents a single chemical bond and/or;

(b) reacting a compound of formula (I) in which Z represents:

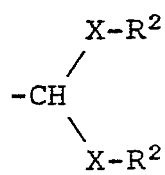

and A and B taken together represent a single bond, with a thiol of formula $R^1SH$ so as to form a compound of formula I in which A represents $SR^1$ and B is hydrogen; and/or

(c) hydrolysing a compound of formula (I) in which Z represents:

$X-R^2$<br>
/<br>
-CH<br>
\\<br>
$X-R^2$

A represents $SR^1$ and B is hydrogen, so as to prepare a compound of formula (I) in which Z is -CHO, A is $SR^1$ and B is hydrogen; and

(d) optionally salifying any compound of formula I produced above so as to form the corresponding pharmacologically-acceptable salt.

8. A veterinary or pharmaceutical formulation which comprises as an active ingredient a compound of formula (I), or a pharmacologically-acceptable salt thereof, as claimed in any one of claims 1 to 6 together with one or more excipients or carriers therefor.

9. A compound of formula (I), or a pharmacologically-acceptable salt thereof, for use in the treatment or prevention of bacterial infections in warm-blooded mammals.